# EUROPÄISCHE PATENTSCHRIFT

(11) **EP 1 252 216 B1**
(45) Veröffentlichungstag und Bekanntmachung des Hinweises auf die Patenterteilung: **30.07.2003**
(21) Anmeldenummer: 00993622.0
(22) Anmeldetag: 12.12.2000
(51) Int. Cl.: C08G 18/48, C08G 18/50, C08G 65/26, C07C 217/04, C08J 9/00

(54) **AMINOORGANISCHE SUBSTANZEN**
AMINO ORGANIC SUBSTANCES
SUBSTANCES AMINO-ORGANIQUES

(30) Priorität: 23.12.1999 DE 19962269
(43) Veröffentlichungstag der Anmeldung: 30.10.2002
(73) Patentinhaber: BASF AKTIENGESELLSCHAFT, 67056 Ludwigshafen (DE)
(72) Erfinder: BÖHME, Peter, 01561 Böhla (DE); GÜTTES, Bernd, 03238 Sallgast (DE); KNORR, Gottfried, 01987 Schwarzheide (DE)
(86) Internationale Anmeldenummer: EP0012576
(87) Internationale Veröffentlichungsnummer: WO01048048

(56) Entgegenhaltungen:
- EP-A- 0 376 602
- US-A- 4 837 244
- US-A- 5 183 687

## Beschreibung

Die Erfindung betrifft aminoorganische Substanzen, die siliziumfrei sind und sich insbesondere für den Einsatz bei der Herstellung von Polyurethanen (PUR) und Polyisocyanuraten (PIR) eignen.

Bei der Herstellung von PUR und PIR werden eine Reihe von Zusatzstoffen benötigt, die die Eigenschaften des Fertigproduktes maßgeblich mit beeinflussen. Bestandteile bei der Herstellung von insbesondere PUR- und PIR-Schaumstoffen sind u.a. Schaumstabilisatoren und oberflächenaktive Verbindungen. Ihre Funktion besteht in der Unterstützung des Mischungsvorganges der Komponenten, in der Kontrolle der Zellgröße und der Stabilisierung der Zellen während des Schäum- und Polymerisationsprozesses. Herkömmlicherweise werden Schaumstabilisatoren verwendet, die auf der Basis von Siloxanderivaten hergestellt wurden. Ein Nachteil dieser Verbindungsklasse ist einerseits der relativ hohe Preis. Weiterhin gibt es Anhaltspunkte dafür, dass Si-haltige Verbindungen die Brennbarkeit von PUR-Hartschäumen begünstigen. Eine Substitution der Siloxanstabilisatoren durch Si-freie Verbindungen würde also zur Kostenreduzierung beitragen und gleichzeitig den Bedarf an Flammschutzzusätzen im System senken.

Si-freie Schaumstabilisatoren wurden bereits mehrfach in der Patentliteratur erwähnt. Neben einfachen Tensiden, z.B. alkoxylierten Derivaten des Nonylphenols (US-A-5236961), alkoxylierten Fettalkoholen (EP-A-0343463), Fettsäureestern und -amiden (DE-A-19521351), wurden auch Polyetherole beschrieben, deren schaumstabiliserende Eigenschaften auf einem erhöhten Anteil an Butylenglykol und höheren Glykolen beruhen soll (WO 9516721). Als Starter werden OH-Verbindungen verschiedener Funktionalität, aber auch Amine (Ethylendiamin, Ammoniak und Ethanolamin) genannt.

Ebenfalls Si-frei sind Stabilisatoren auf der Basis partiell oder vollständig fluorierter Verbindungen, wie z.B. in US-A-4356273 beschrieben. Nach einem ähnlichen Patent (JP 055723) werden Polyol- und Perfluorteil des Moleküls jeweils über Ether-, Amin-, Amid- oder Sulfonamidgruppen gekoppelt.

In WO 97/14730 werden Si-freie PUR-Hartschäume beschrieben, die allerdings auf der Basis bereits bekannter Si-freier Schaumstabilisatoren hergestellt werden.

Aufgabe der vorliegenden Erfindung war somit die Bereitstellung von Si-freien aminoorganische Substanzen, die sich für den Einsatz als Zusatzstoff bei der Herstellung von PUR und PIR, insbesondere als Schaumstabilisatoren für PUR- und PIR-Hartschäume, eignen.

Die Aufgabe wurde erfindungsgemäß gelöst durch die Zurverfügungstellung von aminoorganischen Substanzen der allgemeinen Formel

(CₒBₙAₘ) -N-R'-N- (AₘBₙCₒ) (F1),

wobei n, m und o ganze natürliche Zahlen darstellen, für die gilt m + n + o = 2, und die Substituenten folgende Strukturen besitzen

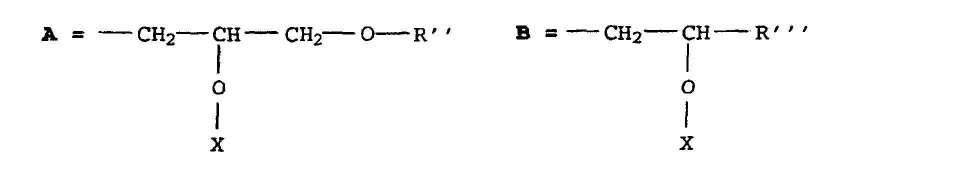

C = ―R'―N―(AₘBₙ)

und darin die Substituenten R', R'' und R''' folgende chemische Zusammensetzung
R' = Alkyl-, Cycloalkyl-
R" = Alkyl-, Cycloalkyl-, Alkylen-, Alkyldien-, Alkylin-, Phenyl-, Mono- und Polyfluoralkylgruppen
R'" = (CH₂)_{z}-CH₃ mit z = 2-16
haben, wobei X eine Polyalkyletherkette ist.

Gegenstände der Erfindung sind demzufolge aminoorganische Substanzen der genannten allgemeinen Formel F1 sowie ein Verfahren zur Herstellung dieser aminoorganischen Substanzen durch Umsetzung von polyfunktionellen Aminoverbindungen der allgemeinen Formel

HₚZᵣ - N - R' - N - ZᵣHₚ (F2)

R' = Alkyl-, Cycloalkylgruppe
Z = - R' - NHₚZᵣ
p und r = 0, 1, 2 und p + r = 2
mit substituierten 2,3-Epoxipropylethern der allgemeinen Struktur 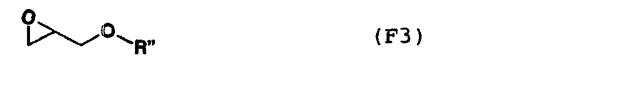
R'' = Alkyl-, Cycloalkyl-, Alkylen-, Alkyldien-, Alkylin-, Phenyl-, Mono- und Polyfluoralkylgruppen
und/oder Alkylepoxiden der allgemeinen Struktur

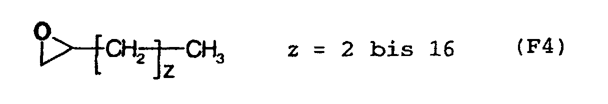

Gegenstände der Erfindung sind weiterhin die Verwendung der aminoorganischen Substanzen als Zusatzstoffe bei der PUR- und PIR-Herstellung, insbesondere als Schaumstabilisatoren bei der PUR- und PIR-Schaumherstellung, sowie die entsprechenden Schaumstabilisatoren selbst.

Die erfindungsgemäßen aminoorganischen Substanzen haben den Vorteil, dass sie insbesondere als speziell entwickelte und maßgeschneiderte Schaumstabilisatoren für hochbelastbare und spezielle PUR- und PIR-Schäume einsetzbar sind. PUR- und PIR-Hartschäume, die unter Einsatz der neuartigen, Si-freien Schaumstabilisatoren hergestellt werden, besitzen eine einheitliche und feine Zellstruktur. Die so erhaltenen Schaumstoffe zeigen keinerlei nachteilige mechanische Eigenschaften im Vergleich zu Schäumen, die unter Verwendung von herkömmlichen Siloxanstabilisatoren hergestellt wurden.

Neu an den in diesem Patent beschriebenen Stabilisatoren ist die Möglichkeit, die für die Stabilisatorfähigkeit verantwortlichen Eigenschaften der Verbindungen in einem relativ weiten Rahmen variieren zu können. Dadurch ergibt sich die Möglichkeit, spezielle Stabilisatoren nach einer Art "Baukastenprinzip" für das jeweilige PUR- oder PIR-System herzustellen.

Die Substanzen mischen sich sehr gut mit den übrigen PUR- bzw. PIR-Komponenten, sind mit den Polyolen verträglich und es sind keinerlei Nebenreaktionen und Produktschädigungen nachweisbar.

Zu den erfindungsgemäßen aminoorganischen Substanzen ist im Einzelnen Folgendes auszuführen:

Sie weisen eine Struktur, gekennzeichnet durch die allgemeine Formel

(CₒBₙAₘ)-N-R'-N-(AₘBₙCₒ) (F1),

auf.

Darin stellen n, m und o ganze natürliche Zahlen dar, für die gilt m + n + o = 2. Vorteilhafterweise wird die Verteilung der Substituenten A, B und C so vorgenommen, daß m + o = 2 bzw. n + o = 2, besonders bevorzugt m = o = 1 und n = o = 1, ergeben.

Die Substituenten weisen dabei folgende Strukturen auf: R' ist erfindungsgemäß eine Alkyl- oder Cycloalkylgruppe. Vorzugsweise stellt R' eine Alkylgruppe mit 2 bis 8 C-Atomen, insbesondere 3 bis 5 C-Atomen, in linearer Anordnung dar. Beispielsweise stellt R' eine n-Butyl- oder tertiäre Butylgruppe dar.

Die Substituenten A, B und C weisen erfindungsgemäß Strukturen der folgenden allgemeinen Formeln auf:

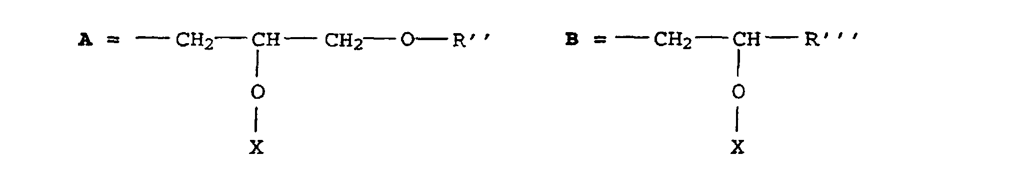

C = ―R'―N―(AₘBₙ)

Der Substituent R'' ist dabei eine Alkyl-, Cycloalkyl-, Alkylen-, Alkyldien-, Alkylin-, Phenyl-, Mono- oder Polyfluoralkylgruppe. R'' stellt vorzugsweise eine Alkylgruppe mit 2 bis 8 C-Atomen, insbesondere 3 bis 4 C-Atomen, dar, wobei die Alkylgruppe sowohl linear, als auch über eine oder zwei sekundäre Alkylgruppen oder eine tertiäre Alkylgruppe verzweigt sein kann.

Der Substituent R''' ist eine CH₃-(CH₂)_{z}-Gruppe mit z = 2 - 16. R''' stellt vorzugsweise eine Alkylgruppe mit 3 bis 17 C-Atomen dar, wobei die Alkylgruppe sowohl linear, als auch über eine oder zwei sekundäre Alkylgruppen oder eine tertiäre Alkylgruppe verzweigt sein kann. Insbesondere weist R''' 3 bis 10 C-Atome in linearer Anordnung auf. Besonders bevorzugt ist R''' einen Alkylrest mit 3 bis 5 C-Atomen.

Die Substituenten R', A und B sowie n und m genügen den oben genannten Definitionen. Sie können gleich oder unterschiedlich zu den entsprechenden Substituenten in F1 sein.

Der Substituent X besteht aus Polyalkyletherketten, die aus Ethylenoxid (EO), Propylenoxid (PO) und Butylenoxid (BO) zusammengesetzt sind. Sie können hergestellt werden durch Addition von EO, PO und/oder BO an die Hydroxylgruppe und durch die allgemeine Formel

X = (EO)ₐ(PO)_{b}(BO)_{c}

charakterisiert werden, wobei a, b und c ganze natürliche Zahlen sind, für die gilt a + b + c = 2 bis 40, vorzugsweise 2 bis 20, besonders bevorzugt 5 bis 12. In der Polyalkyletherkette können die Alkylenoxyide sowohl als Blöcke, als auch statistisch vorliegen. Die Polyalkyletherkette kann sowohl ein Homopolymer jedes der drei Monomeren oder aber auch ein Co- bzw. Triblockcopolymer sein.

Vorzugsweise liegt der Anteil an PO in der Kette zwischen 50 und 100 Masse-% und der an EO und/oder BO zwischen 0 und 50 Masse-%.

Insbesondere zeigen die Verbindungen der Struktur D₂-N-CH₂-CH₂-N-D₂ mit D = -CH₂-CH₂-CH₂-N-A₂ interessante Anwendungseigenschaften. Als R'' können hierbei sowohl aliphatische als auch cyclische Kohlenwasserstoffe, sowie aromatische Gruppierungen Verwendung finden, bevorzugt eingesetzt werden Alkylgruppen mit mehr als zwei CH₂-Gruppen, deren Struktur sowohl geradkettig als auch verzweigt sein kann.

Die erfindungsgemäßen aminoorganischen Substanzen weisen vorzugsweise eine OH-Zahl von 20 bis 700 mg KOH/g, besonders bevorzugt von 50 bis 200 mg KOH/g, und einen Gehalt an tertiär substituiertem Stickstoff von größer als 95 % des Gesamtstickstoffgehaltes auf.

Die Herstellbarkeit der erfindungsgemäßen aminoorganischen Substanzen ist beispielsweise durch Umsetzung von polyfunktionellen Aminoverbindungen der allgemeinen Formel

HₚZᵣ - N - R' - N - ZᵣHₚ (F2)

R' = Alkyl-, Cycloalkylgruppe
Z = - R' - NHₚZᵣ
p und r = 0, 1, 2 und p + r = 2
mit substituierten 2,3-Epoxipropylethern der allgemeinen Struktur 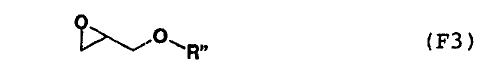
R'' = Alkyl-, Cycloalkyl-, Alkylen-, Alkyldien-, Alkylin-, Phenyl-, Mono- und Polyfluoralkylgruppen
und/oder Alkylepoxiden der allgemeinen Struktur 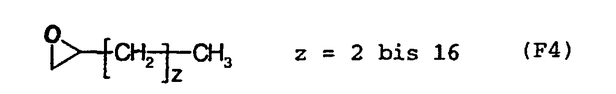 problemlos möglich.

Vorzugsweise werden als polyfunktionelle Aminoverbindungen lineare Alkylenpolyamine eingesetzt. Besonders bevorzugt ist N,N'-bis(3-aminopropyl)-ethylendiamin.

Als substituierte 2,3-Epoxipropylether werden vorzugsweise Verbindungen, die Alkylketten mit 3 bis 5 C-Atomen enthalten, eingesetzt. Besonders bevorzugt werden n- und tert.-Butylderivate verwendet.

Als substituierte Alkylepoxide werden vorzugsweise Verbindungen mit 3 bis 5 C-Atomen verwendet.

Die Herstellung der erfindungsgemäßen aminoorganischen Substanzen wird vorteilhafterweise so durchgeführt, daß die polyfunktionellen Aminoverbindungen der allgemeinen Formel F2 in einem ersten Schritt mit substituierten 2,3-Epoxipropylethern der allgemeinen Struktur F3 und/oder mit Alkylepoxiden der allgemeinen Struktur F4 umgesetzt werden und das dadurch entstehende Zwischenprodukt in einer nachfolgenden Alkoxylierung mit den Monomeren EO, PO und/oder BO als Homo-, Blockco- oder Blocktripolymerisate hergestellt wird.

Die erfindungsgemäßen aminoorganischen Substanzen können durch Variation der Ausgangsstoffe und Verfahrensbedingungen vorteilhafterweise maßgeschneidert werden. Sie können damit für die weiter unten beispielhaft beschriebenen Einsatzzwecke speziell entwickelt und angepaßt werden, um ganz gezielte Eigenschaften der mit diesen Produkten hergestellten PUR und PIR, wie beispielsweise hochbelastbare PUR- und PIR-Schäume, zu erhalten.

Die erfindungsgemäßen aminoorganischen Verbindungen können als Zusatzstoffe bei der PUR- und PIR-Herstellung eingesetzt werden. Als besonders vorteilhaft hat sich ihre Verwendung als Schaumstabilisatoren bei der PUR- und PIR-Schaumherstellung erwiesen.

Die Verbindungen können entweder als Einzelsubstanzen oder aber auch als Gemisch untereinander, mit weiteren Si-freien Schaumstabilisatoren und/oder mit Si-haltigen Substanzen, eingesetzt werden. Dabei können entsprechend der zu stabilisierenden Schaumart die Substituenten und Kettenlängen sowie die Gemischanteile modifiziert und variiert werden. Die erfindungsgemäßen aminoorganischen Substanzen stellen somit hochaktive, maßgeschneiderte Schaumstabilisatoren insbesondere für PUR und PIR-Schäume dar.

Es ist auch deren Einsatz als oberflächenaktive Substanz, als Zellöffner, als Kettenverlängerer, als Vernetzersubstanz und/oder als weiterer Hilfs- und Zusatzstoff für PUR- und PIR-Schäume bzw. auch für andere Anwendungen möglich.

Die Substanzen mischen sich sehr gut mit den übrigen PUR- bzw. PIR-Komponenten, sind mit den Polyolen verträglich und es sind keinerlei Nebenreaktionen und Produktschädigungen nachweisbar.

PUR- und PIR-Hartschäume, die unter Einsatz der neuartigen, Si-freien Schaumstabilisatoren hergestellt werden, besitzen eine einheitliche und feine Zellstruktur. Die so erhaltenen Schaumstoffe zeigen keinerlei nachteilige mechanische Eigenschaften im Vergleich zu Schäumen, die unter Verwendung von herkömmlichen Siloxanstabilisatoren hergestellt wurden.

Beispielhaft kann eine erfindungsgemäße aminoorganische Verbindung folgendermaßen synthetisiert werden:

### Beispiel 1

Im ersten Syntheseschritt wurde N,N'-bis(3-aminopropyl)-ethylendiamin (N4-Amin) mit 0,1 Masse-% Kaliumhydroxid als Katalysator versetzt und auf 70°C erwärmt. Danach wurde mit trockenem Stickstoff inertisiert und unter kräftigem Rühren die sechsfache molare Menge an tertiärem Butylglycidether zugegeben. Es erfolgte eine Nachreaktion bei 70°C über 5 Stunden. Danach wurde mit HCl neutralisiert, der Niederschlag abfiltriert und das Lösungsmittel im Vakuum abgezogen. Erhalten wurde eine hellgelbe, hochviskose Flüssigkeit.

Das Produkt besaß eine OH-Zahl von 360 mg KOH/g, einen pH-Wert von 10,5 und einen Gehalt an tertiärem Amin von größer 98 %. Im NMR-Spektrum (Lösungsmittel DMSO-d₆) bewiesen die CH₃-Signale (¹H = 1,12 ppm, ¹³C = 27,3 ppm) die Anwesenheit der tertiären Butylgruppe. Im IR-Spektrum wurden neben der charakteristischen CH₃-Valenzschwingungsbande (2973 cm⁻¹) der tertiären Butylgruppe auch die OH-Gruppen bei 3425 cm⁻¹ nachgewiesen.

Die so erhaltene Zwischenverbindung wurde danach mit PO umgesetzt. Dazu wurde die Substanz mit Kaliumhydroxid als Katalysator versetzt (0,1 bis 0,3 Masse-%) und unter Inertgasatmosphäre mit PO bei einer Temperatur von 115°C umgesetzt (Rührgeschwindigkeit 500 U/min, Dosiergeschwindigkeit 1 bis 2,5 kg/h, max. Druck 7 bar). Die Aufarbeitung erfolgte durch Neutralisation mit Ionenaustauschermaterialien (Ambosol), Filtration und eine Vakuumbehandlung zur Abtrennung leichtflüchtiger Nebenprodukte. Das so erhaltene Produkt war eine leicht gelblich gefärbte, klare und niedrigviskose Flüssigkeit. Die OH-Zahl betrug 76 mg KOH/g, die Säurezahl max. 0,01 mg KOH/g, der pH-Wert 10,3, die Viskosität (25°C) 920 mPa s, der Wassergehalt war kleiner 0,05 Gew.-% und der Kaliumgehalt lag unter 5 ppm.

Die so hergestellte Verbindung kann durch folgende Strukturformel dargestellt werden:

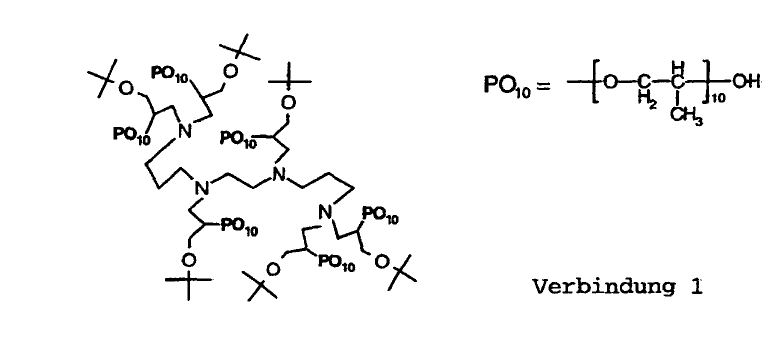

### Beispiel 2 (Vergleich) :

### A-Komponente

Mischung aus
- 41,2: Gew.-Teilen eines Polyetherpolyols mit einer OH-Zahl von 400 mg KOH/g, hergestellt durch anionische Polyaddition von PO an Saccharose und Glycerin;
- 12: Gew.-Teilen eines Flammschutzmittels, TCPP (Fa. BASF);
- 27: Gew.-Teilen eines Flammschutzmittels, IXOL B 251 (Fa. Solvay);
- 3,2: Gew.-Teilen Glycerin;
- 1,5: Gew.-Teilen eines Schaumstabilisators auf Silikonbasis (Tegastab B 8409, Fa. Goldschmidt);
- 0,8: Gew.-Teilen N,N-Dimethyl-cyclohexylamin;
- 2,3: Gew.-Teilen Wasser und
- 12: Gew.-Teilen R 141 b.

### B-Komponente

Mischung aus Diphenylmethandiisocyanaten und Polyphenylpolymethylenpolyisocyanaten (Roh-MDI), NCO-Gehalt 31,5 Gew.-%.

Die beschriebenen A- und B-Komponenten wurden in der für die maschinelle Verarbeitung der PUR üblichen Weise mit einem Mischungsverhältnis von A : B = 100 : 123 in eine auf 45°C beheizte Form der Größe 200 cm x 20 cm x 5 cm eingebracht und nach 5 min entformt. Die Dichte des Schaumstoffes betrug 35 kg/m³.

Aus diesem Schaumstoff wurden Prüfkörper der Abmessung 20 cm x 20 cm x 5 cm herausgesägt und an der Oberfläche die Lunkerhäufigkeit mit 10 % bestimmt.

### Beispiel 3 (erfindungsgemäß) :

### A-Komponente

Mischung analog Beispiel 2, aber anstelle des Silikonstabilisators Tegastab B 8409 wurden 1,5 Gew.-Teile der Verbindung aus Beispiel 1 eingesetzt.

### B-Komponente

### Mischung analog Beispiel 2

Die Verarbeitung zu einem Schaumstoff und die Herstellung der Prüfkörper erfolgte ebenfalls analog Beispiel 2. An der Oberfläche wurde die Lunkerhäufigkeit mit 8 % bestimmt.

## Patentansprüche

1. Aminoorganische Substanzen der allgemeinen Formel
(CₒBₙAₘ)-N-R'-N-(AₘBₙCₒ) (F1),
wobei m, n und o ganze natürliche Zahlen darstellen, für die gilt m + n + o = 2, und die Substituenten folgende Strukturen besitzen 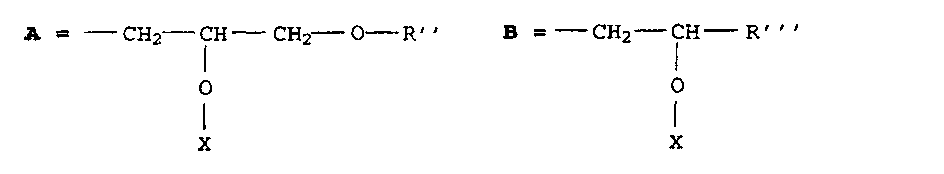
C = ―R'―N―(AₘBₙ)
und darin die Substituenten R', R'' und R''' folgende chemische Zusammensetzung
R' = Alkyl-, Cycloalkyl-
R" = Alkyl-, Cycloalkyl-, Alkylen-, Alkyldien-, Alkylin-, Phenyl-, Mono- und Polyfluoralkylgruppen
R'''= (CH₂)_{z}-CH₃ mit z = 2-16
haben, wobei X eine Polyalkyletherkette ist.

2. Aminoorganische Substanzen gemäß Anspruch 1, **dadurch gekennzeichnet, dass** R' eine Alkylgruppe mit 2 bis 8 C-Atomen in linearer Anordnung ist.

3. Aminoorganische Substanzen gemäß den Ansprüche 1 oder 2, **dadurch gekennzeichnet, dass** für die Verteilung der Substituenten A, B und C gilt m + o = 2 bzw. n + o = 2 und bevorzugt m = o = 1 und n = o = 1.

4. Aminoorganische Substanzen gemäß den Ansprüchen 1 bis 3 **dadurch gekennzeichnet, dass** A als Substituenten R'' eine Alkylgruppe mit 2 bis 8 C-Atomen enthält, wobei die Alkylgruppe sowohl linear, als auch über eine oder zwei sekundäre oder eine tertiäre Alkylgruppe verzweigt sein kann.

5. Aminoorganische Substanzen gemäß einem der Ansprüche 1 bis 4, **dadurch gekennzeichnet, dass** B als Substituenten R''' eine Alkylgruppe mit 3 bis 17 C-Atomen enthält, wobei die Alkylgruppe sowohl linear, als auch über eine oder zwei sekundäre Alkylgruppen oder eine tertiäre Alkylgruppe verzweigt sein kann.

6. Aminoorganische Substanzen gemäß einem der Ansprüche 1 bis 5, **dadurch gekennzeichnet, dass** in C die Substituenten R', A und B sowie n und m den Ansprüchen 1 bis 5 genügen und gleich oder unterschiedlich zu den entsprechenden Substituenten in F1 sein können.

7. Aminoorganische Substanzen gemäß einem der Ansprüche 1 bis 6, **dadurch gekennzeichnet, dass** der Substituent X aus einer Polyalkyletherkette, hergestellt durch Addition von insgesamt 2 bis 40 Molekülen Ethylen-, Propylen- und/oder Butylenoxid an die Hydroxylgruppe, besteht, in der die Alkylenoxide sowohl als Blöcke, als auch statistisch verteilt vorliegen können.

8. Aminoorganische Substanzen gemäß einem der Ansprüche 1 bis 7, **dadurch gekennzeichnet, dass** die Polyalkyletherkette insgesamt 2 bis 20 Moleküle Ethylen-, Propylen- und/oder Butylenoxid enthält.

9. Aminoorganische Substanzen gemäß einem der Ansprüche 1 bis 8, **dadurch gekennzeichnet, dass** der Anteil an Propylenoxid zwischen 50 und 100 Masse-% und der an Ethylenoxid und/oder Butylenoxid zwischen 0 und 50 Masse-% liegt.

10. Aminoorganische Substanzen gemäß einem der Ansprüche 1 bis 9, **dadurch gekennzeichnet, dass** sie eine OH-Zahl von 20 bis 700 mg KOH/g Polyetherol und einen Gehalt an tertiär substituiertem Stickstoff von größer 95 % des Gesamtstickstoffgehaltes aufweisen.

11. Aminoorganische Substanzen gemäß einem der Ansprüche 1 bis 10, herstellbar durch Umsetzung von polyfunktionellen Aminoverbindungen der allgemeinen Formel
HₚZᵣ - N - R' - N - ZᵣHₚ (F2)
R' = Alkyl-, Cycloalkylgruppe
Z = - R' - NHₚZᵣ
p und r = 0, 1, 2 und p + r = 2
mit substituierten 2,3-Epoxipropylethern der allgemeinen Struktur 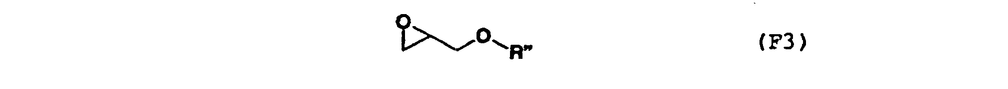
R'' = Alkyl-, Cycloalkyl-, Alkylen-, Alkyldien-, Alkylin-, Phenyl-, Mono- und Polyfluoralkylgruppen
und/oder Alkylepoxiden der allgemeinen Struktur

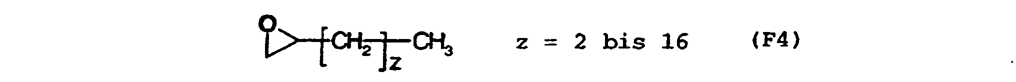

12. Verfahren zur Herstellung der aminoorganischen Substanzen gemäß einem der Ansprüche 1 bis 11, **dadurch gekennzeichnet, dass** polyfunktionelle Aminoverbindungen der allgemeinen Formel F2 in einem ersten Schritt mit den substituierten 2,3-Epoxipropylethern der allgemeinen Struktur F3 und/oder mit Alkylepoxidverbindung der allgemeinen Struktur F4 umgesetzt werden und das dadurch entstehende Zwischenprodukt in einer nachfolgenden Alkoxylierungsreaktion mit den Monomeren Ethylen-, Propylen- und/oder Butylenoxid umgesetzt wird.

13. Verwendung der aminoorganischen Substanzen gemäß einem der Ansprüche 1 bis 11 als Zusatzstoffe bei der Polyurethan- und Polyisocyanuratherstellung.

14. Verwendung der aminoorganischen Substanzen gemäß einem der Ansprüche 1 bis 11 als Schaumstabilisatoren bei der Polyurethan- und Polyisocyanuratschaumherstellung.

15. Schaumstabilisatoren, **dadurch gekennzeichnet, dass** sie aminoorganische Substanzen gemäß einem der Ansprüche 1 bis 10 sind.

16. Schaumstabilisatoren, **dadurch gekennzeichnet, dass** sie gemäß Anspruch 11 oder 12 herstellbar sind.

## Claims

1. An aminoorganic substance of the formula
(CₒBₙAₘ)―N―R'―N―(AₘBₙCₒ) (F1),
where m, n and o are natural numbers which obey the relationship m + n + o = 2, and the substituents have the following structures 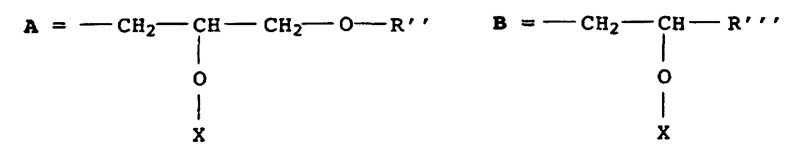
C = ―R'―N―(AₘBₙ)
in which the substituents R', R'' and R''' have the following chemical compositions
R' = alkyl, cycloalkyl,
R" = alkyl, cycloalkyl, alkenyl, alkadienyl, alkynyl, phenyl, monofluoroalkyl or polyfluoroalkyl,
R'''= (CH₂)_{z}-CH₃ where z = 2-16,
and X is a polyalkyl ether chain.

2. An aminoorganic substance as claimed in claim 1, wherein R' is a linear alkyl having from 2 to 8 carbon atoms.

3. An aminoorganic substance as claimed in claim 1 or 2, wherein the substituents A, B and C are distributed such that m + o = 2 or n + o = 2 and preferably m = o = 1 and n = o = 1.

4. An aminoorganic substance as claimed in any of claims 1 to 3, wherein the substituent R'' in A is an alkyl group having from 2 to 8 carbon atoms which may be either linear or branched via one or two secondary carbon atoms or one tertiary carbon atom.

5. An aminoorganic substance as claimed in any of claims 1 to 4, wherein the substituent R''' in B is an alkyl group having from 3 to 17 carbon atoms which may be either linear or branched via one or two secondary carbon atoms or one tertiary carbon atom.

6. An aminoorganic substance as claimed in any of claims 1 to 5, wherein the substituents R', A and B and the indices n and m in C are as defined in any of claims 1 to 5 and may be identical to or different from the corresponding substituents in F1.

7. An aminoorganic substance as claimed in any of claims 1 to 6, wherein the substituent X comprises a polyalkyl ether chain prepared by addition of a total of from 2 to 40 molecules of ethylene oxide, propylene oxide and/or butylene oxide onto the hydroxyl group, where the alkylene oxides can either be present as blocks or be randomly distributed.

8. An aminoorganic substance as claimed in any of claims 1 to 7, wherein the polyalkyl ether chain comprises a total of from 2 to 20 molecules of ethylene oxide, propylene oxide and/or butylene oxide.

9. An aminoorganic substance as claimed in any of claims 1 to 8, wherein the proportion of propylene oxide is from 50 to 100% by mass and that of ethylene oxide and/or butylene oxide is from 0 to 50% by mass.

10. An aminoorganic substance as claimed in any of claims 1 to 9 which has an OH number of from 20 to 700 mg KOH/g of polyetherol and has a content of tertiary nitrogen of greater than 95% of the total nitrogen content.

11. An aminoorganic substance as claimed in any of claims 1 to 10 which can be prepared by reacting polyfunctional amino compounds of the formula
HₚZᵣ - N - R' - N - ZᵣHₚ (F2)
R' = alkyl, cycloalkyl
Z = - R' - NHₚZᵣ
p and r = 0, 1, 2 and p + r = 2
with substituted 2,3-epoxypropyl ethers having the structure 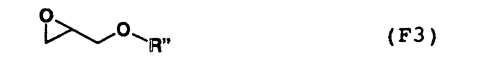
R'' = alkyl, cycloalkyl, alkenyl, alkadienyl, alkynyl, phenyl, monofluoroalkyl or polyfluoroalkyl,
and/or alkyl epoxides having the structure

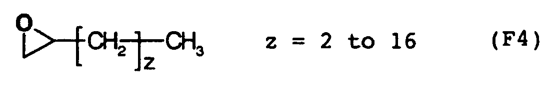

12. A process for preparing aminoorganic substances as claimed in any of claims 1 to 11, which comprises reacting polyfunctional amino compounds of the formula F2 with substituted 2,3-epoxypropyl ethers having the structure F3 and/or with alkyl epoxide compounds having the structure F4 in a first step and reacting the resulting intermediate with the monomers ethylene oxide, propylene oxide and/or butylene oxide in a subsequent alkoxylation reaction.

13. The use of an aminoorganic substance as claimed in any of claims 1 to 11 as additive in polyurethane and polyisocyanurate production.

14. The use of an aminoorganic substance as claimed in any of claims 1 to 11 as foam stabilizers in polyurethane and polyisocyanurate production.

15. A foam stabilizer which is an aminoorganic substance as claimed in any of claims 1 to 10.

16. A foam stabilizer which can be prepared as set forth in claim 11 or 12.

## Revendications

1. Substances amino-organiques répondant à la formule générale
(CₒBₙAₘ)-N-R'-N-(AₘBₙCₒ) (F1),
dans laquelle m, n et o représentent des nombres naturels entiers, de telle sorte que m + n + o = 2, et les substituants possèdent les structures ci-après : 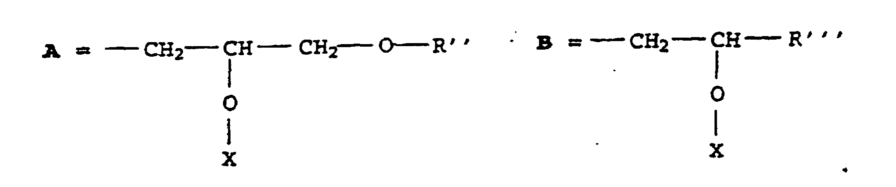
C = ―R'―N―(AₘBₙ)
et dans ces dernières, les substituants R', R" et R"' possèdent la composition chimique ci-après :
R' représente un groupe alkyle, un groupe cycloalkyle,
R" représente un groupe alkyle, un groupe cycloalkyle, un groupe alkylène, un groupe alkyldiène, un groupe alcynyle, un groupe phényle, un groupe monofluoroalkyle et un groupe polyfluoroalkyle,
R''' représente un groupe (CH₂)_{z}-CH₃ où z = 2 - 16,
X représentant une chaîne de polyalkyléther.

2. Substances amino-organiques selon la revendication 1, **caractérisées en ce que** R' représente un groupe alkyle contenant de 2 à 8 atomes de carbone dans un agencement linéaire.

3. Substances amino-organiques selon la revendication 1 ou 2, **caractérisées en ce que** la répartition des substituants A, B et C est telle que m + o = 2, respectivement n + o = 2, et de préférence m = o = 1 et n = o = 1.

4. Substances amino-organiques selon les revendications 1 à 3, **caractérisées en ce que** A contient, à titre de substituant R", un groupe alkyle contenant de 2 à 8 atomes de carbone, le groupe alkyle pouvant être aussi bien linéaire que ramifié via un ou deux groupes alkyle secondaires ou via un groupe alkyle tertiaire.

5. Substances amino-organiques selon l'une quelconque des revendications 1 à 4, **caractérisées en ce que** B contient, à titre de substituant R"', un groupe alkyle contenant de 3 à 17 atomes de carbone, le groupe alkyle pouvant être aussi bien linéaire que ramifié via un ou deux groupes alkyle secondaires ou via un groupe alkyle tertiaire.

6. Substances amino-organiques selon l'une quelconque des revendications 1 à 5, **caractérisées en ce que**, dans C, les substituants R', A et B, ainsi que n et m répondent aux revendications 1 à 5 et peuvent être identiques ou différents aux substituants correspondants dans F1.

7. Substances amino-organiques selon l'une quelconque des revendications 1 à 6, **caractérisées en ce que** le substituant X est constitué d'une chaîne de polyalkyléther, préparée par addition d'un total de 2 à 40 molécules d'oxyde d'éthylène, d'oxyde de propylène et/ou d'oxyde de butylène au groupe hydroxyle, dans laquelle les oxydes d'alkylènes peuvent aussi bien être présents sous la forme de séquences que sous la forme d'une répartition statistique.

8. Substances amino-organiques selon l'une quelconque des revendications 1 à 7, **caractérisées en ce que** la chaîne de polyalkyléther contient au total de 2 à 20 molécules d'oxyde d'éthylène, d'oxyde de propylène et/ou d'oxyde de butylène.

9. Substances amino-organiques selon l'une quelconque des revendications 1 à 8, **caractérisées en ce que** la fraction d'oxyde de propylène représente entre 50 et 100 % en masse et la fraction d'oxyde d'éthylène et/ou d'oxyde de butylène représente entre 0 et 50 % en masse.

10. Substances amino-organiques selon l'une quelconque des revendications 1 à 9, **caractérisées en ce qu'**elles présentent un indice OH de 20 à 700 mg de KOH/g de polyétherol et une teneur en azote trisubstitué supérieure à 95 % de la teneur totale en azote.

11. Substances amino-organiques selon l'une quelconque des revendications 1 à 10, que l'on peut préparer par une mise en réaction de composés amino polyfonctionnels répondant à la formule générale
HₚZᵣ-N-R'-N-ZᵣHₚ (F2)
dans laquelle
R' représente un groupe alkyle, un groupe cycloalkyle
Z représente un groupe - R' - NHₚZᵣ
p et r = 0, 1, 2 et p + r = 2
avec des 2,3-époxypropyléthers substitués possédant la structure générale 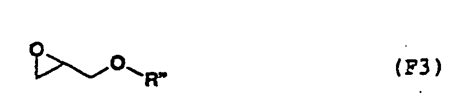 dans laquelle R" représente un groupe alkyle, un groupe cycloalkyle, un groupe alkylène, un groupe alkyldiène, un groupe alcynyle, un groupe monofluoroalkyle et un groupe polyfluoroalkyle,
et/ou avec des alkylépoxydes possédant la structure générale

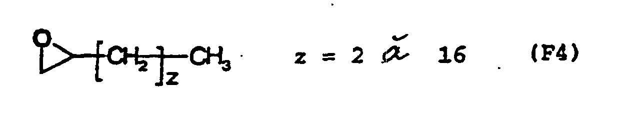

12. Procédé pour la préparation des substances amino-organiques selon l'une quelconque des revendications 1 à 11, **caractérisé en ce qu'**on fait réagir des composés amino polyfonctionnels répondant à la formule générale F2 dans une première étape avec les 2,3-époxypropyléthers substitués possédant la structure générale F3 et/ou avec le composé d'alkylépoxyde possédant la structure générale F4 et on fait réagir le produit intermédiaire ainsi obtenu dans une réaction d'alcoxylation ultérieure avec de l'oxyde d'éthylène, de l'oxyde de propylène et/ou de l'oxyde d'éthylène, à titre de monomères.

13. Utilisation des substances amino-organiques selon l'une quelconque des revendications 1 à 11, à titre d'additifs lors de la préparation de polyuréthane et de polyisocyanurate.

14. Utilisation des substances amino-organiques selon l'une quelconque des revendications 1 à 11, à titre de stabilisateurs de mousses lors de la préparation de mousse de polyuréthane et de polyisocyanurate.

15. Stabilisateurs de mousses, **caractérisés en ce qu'**il s'agit des substances amino-organiques selon l'une quelconque des revendications 1 à 10.

16. Stabilisateurs de mousses, **caractérisés en ce qu'**on peut les préparer conformément à la revendication 11 ou 12.
